# EUROPEAN PATENT APPLICATION

(11) **EP 2 127 601 A1**
(43) Date of publication of application: **02.12.2009**
(21) Application number: 09159889.6
(22) Date of filing: 11.05.2009
(51) Int. Cl.: A61B 8/08, A61M 5/168

(54) **Extravasation detection in an ultrasound system**

(30) Priority: 28.05.2008 KR 20080049379
(71) Applicant: MEDISON CO., LTD., Nam-myun Hongchun-gun Kangwon do 250-870 (KR)
(72) Inventor: Hyun, Dong Gyu, Seoul 135-851 (KR); Yang, Eun Ho, Seoul 135-851 (KR)
(74) Representative: Schmid, Wolfgang

(57) **Abstract**

Embodiments for detecting extravasation in an ultrasound system are disclosed. The ultrasound system includes a Tx/Rx unit to transmit an ultrasound beam to a target object and receive ultrasound echoes reflected from the target object to thereby output receive signals. The receive signals include first receive signals obtained before injecting a fluid into the target object and second receive signals obtained after injecting the fluid into the target object. The ultrasound system further includes a processing unit to form a first ultrasound image based on the first receive signals and a second ultrasound image based on the second receive signal. The processing unit further compares the first ultrasound image and the second ultrasound image for detecting extravasation at the target object.

## Description

The present application claims priority from Korean Patent Application No. 10-2008-0049379 filed on May 28, 2008, the entire subject matter of which is incorporated herein by reference.

### TECHNICAL FIELD

The present disclosure relates to ultrasound systems, and more particularly to an ultrasound system and method of detecting extravasation caused by the injection of a contrast agent into a blood vessel.

### BACKGROUND

Surgical treatment using a medical needle such as ablator or biopsy has recently become popular due to relatively small incisions required in such a procedure. The surgical treatment is performed by inserting the medical needle into an internal region of a human body while referring to an internal image of the human body. Such surgical treatment, which is performed while observing internal organs of the human body with the help of a diagnostic imaging system, is referred to as an interventional treatment. The interventional treatment is performed by directing the medical needle to the lesion to be treated or examined with reference to images during the treatment. The images are acquired by employing a computerized tomography (CT) scanner generally used in a radiology department or a magnetic resonance imaging (MRI) system. Compared to a normal surgical treatment requiring relatively wide incisions to open the lesion, the interventional treatment has the advantages of low costs and producing effective operation results. This is because general anesthesia is not necessary for the interventional treatment and patients are subjected to less pain while benefiting from rapid recovery.

In CT, a contrast agent is generally injected into the blood to image a blood vessel. In such a case, if the needle is incorrectly inserted into the blood vessel or an injection dose of the contrast agent is inaccurately controlled, then the contrast agent may flow out from the blood vessel to neighboring tissues. This damages the cells or destroys the tissues, which is referred to as an "extravasation." The extravasation may occur due to an intravenous injection as well as the injection of the contrast agent. The extravasation may cause serious tissue necrosis. As such, it is important to detect it at an early stage. In order to detect the extravasation, a method using a change in an electrical resistance at a patient flesh or a method using a change in a flesh temperature has been researched and developed in the art.

Since the electrical characteristic or temperature of the flesh may be changed according to a motion of the patient, the conventional method may incorrectly detect the extravasation even when the extravasation doe not occur. Also, the method using the electrical characteristic of the skin may be applied only when the flesh swells up.
Thus, it may take a long time to detect the extravasation. Further, the conventional method cannot visually provide the extravasation at the blood vessel.

### SUMMARY

Embodiments for forming an elastic image in an ultrasound system are disclosed herein. In one embodiment, by way of non-limiting example, an ultrasound system comprises:
a transmission/reception (Tx/Rx) unit configured to transmit an ultrasound beam to a target object and receive ultrasound echoes reflected from the target object to thereby output receive signals, wherein the receive signals include first receive signals obtained before injecting a fluid into the target object and second receive signals obtained after injecting the fluid into the target object; and a processing unit configured to form a first ultrasound image based on the first receive signals and a second ultrasound image based on the second receive signal, the processing unit being further configured to compare the first ultrasound image and the second ultrasound image for detecting extravasation at the target object.

In another embodiment, there is provided a method of detecting extravasation in an ultrasound system including a transmission/reception (Tx/Rx) unit and a processing unit, comprising: using a transmission/reception (Tx/Rx) unit within the ultrasound system to transmit an ultrasound beam to a target object and receive ultrasound echoes reflected from the target object to thereby output receive signals, wherein the receive signals include first receive signals obtained before injecting a fluid into the target object and second receive signals obtained after injecting the fluid into the target object; and using a processing unit within the ultrasound system to form a first ultrasound image based on the first receive signals and a second ultrasound image based on the second receive signal, and compare the first ultrasound image and the second ultrasound image for detecting extravasation at the target object.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing one embodiment of a processing unit.
- FIG. 3: is a block diagram showing another embodiment of a processing unit.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

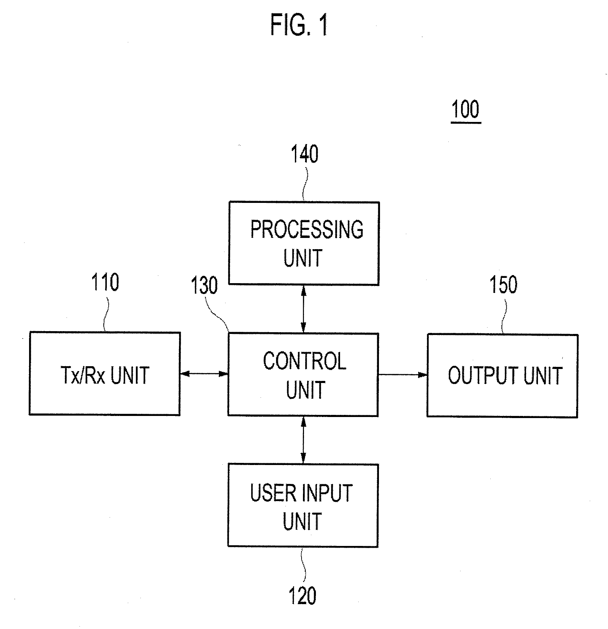

FIG. 1 is a block diagram showing an illustrative embodiment of an ultrasound system 100. As depicted therein, the ultrasound system 100 may include a transmission/reception (Tx/Rx) unit 110 configured to transmit/receive an ultrasound beam to/from a target object to thereby output electrical receive signals. In one embodiment, the target object may include a blood vessel such as a vein. The Tx/Rx unit 110 may include a probe (not shown) to be placed on a surface adjacent to a position where a fluid is injected into the blood vessel. For the sake of convenience, a contrast agent will be referred to as an example of the fluid, which is injected into the blood vessel. However, the fluid is not limited thereto. The fluid may include any type of medications being injected into the blood vessel. The probe may be fixed at a predetermined location by using a fixing unit (not shown).

The ultrasound system 100 may further include a user input unit 120 allowing a user to input an instruction for operating the ultrasound system. The ultrasound system 100 may further include a control unit 130 configured to control the Tx/Rx of the ultrasound beam in the Tx/Rx unit 110 in response to the instruction. The control unit 130 may be operable to control the operation of the Tx/Rx unit 110 in response to the instruction. In one embodiment, the Tx/Rx unit 110 may be controlled to transmit a first ultrasound beam before injecting the contrast agent into the blood vessel and a second ultrasound beam after injecting the contrast agent into the blood vessel. In one embodiment, the Tx/Rx unit 120 may be controlled to output first receive signals in response to the transmission of the first ultrasound beam and second receive signals in response to the transmission of the second ultrasound beam. In another embodiment, the Tx/Rx unit 120 may be controlled to repeatedly transmit the ultrasound beam at a constant interval while injecting the contrast agent. In such a case, the Tx/Rx unit 120 may be controlled to sequentially output a plurality of receive signals in response to the repeated transmission of the ultrasound beam.

The ultrasound system 100 may further include a processing unit 140. The processing unit 140 may be operable to form a plurality of ultrasound images based on the first and second receive signals. The processing unit 140 may be further operable to detect extravasation, which is caused by the injection of the contrast agent, from the ultrasound images.

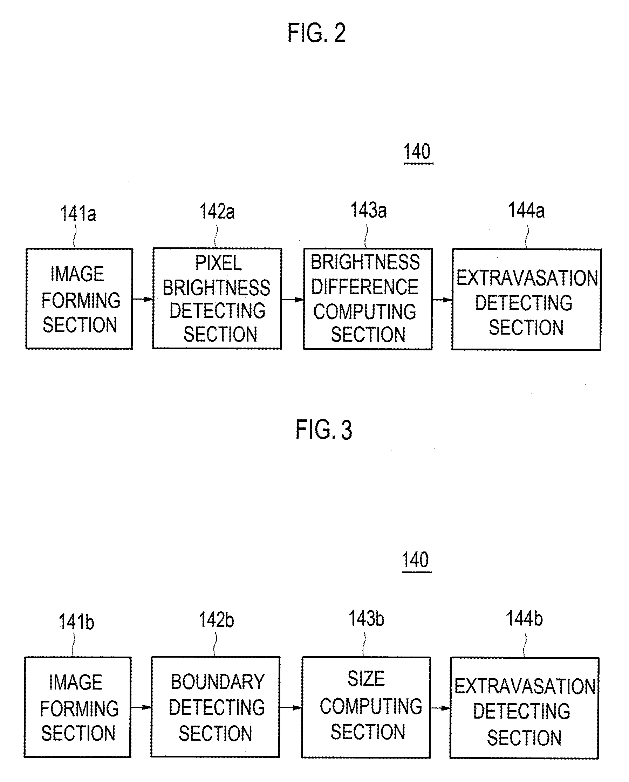

FIG. 2 is a block diagram showing an illustrative embodiment of the processing unit 140. As shown in FIG. 2, the processing unit 140 may include an image forming section 141a. In one embodiment, the image forming section 141a may be operable to form first and second ultrasound images based on the first and second receive signals outputted from the Tx/Rx unit 110. The first ultrasound image may represent an image of the target object obtained before injecting the contrast agent, while the second ultrasound image may represent an image of the target object obtained after injecting the contrast agent. In another embodiment, the image forming section 141a may be configured to form more than two ultrasound images based on multiple receive signals outputted from the Tx/Rx unit 110.

The processing unit 140 may further include a pixel brightness detecting section 142a that may be operable to detect the brightness of each of the pixels contained in the ultrasound images. For example, the pixel brightness detecting section 142a may detect the brightness of each pixel within the first ultrasound image and the second ultrasound image.

The processing unit 140 may further include a brightness difference computing section 143a. The brightness difference computing section 143a may be operable to compute a brightness difference between each pair of pixels taken from the pixels existing in identical locations at the ultrasound images. For example, the brightness difference computing section 143a may be operable to compute a brightness difference between pixels positioned at identical locations at the first ultrasound image and the second ultrasound image. In one embodiment, the brightness difference may be computed by subtracting a brightness value of a corresponding pixel in the second ultrasound image from a brightness value of a pixel in the first ultrasound image.

The processing unit 140 may further include an extravasation detecting section 144a. In one embodiment, the extravasation detecting section 144a may be operable to detect pixels having a brightness difference of a negative value, the absolute value of the brightness difference of which is greater than a first predetermined threshold. For example, a threshold used to detect a cystoma, which is generally represented with lower brightness than that of a normal tissue in an ultrasound image, may be adopted to determine the first predetermined threshold. If the pixel having the brightness difference of a negative value, the absolute value of which is greater than the first predetermined threshold, is detected, then the extravasation detecting section 114a may be operable to output extravasation detection information. In one embodiment, the extravasation detection information may include at least one of icon, text, voice, image, color and alarm, although it is not limited thereto.

In another embodiment, the extravasation detecting section 114a may be operable to detect pixels having a brightness difference of a positive value, the absolute value of which is greater than a second predetermined threshold. A threshold used to detect a necrosis tissue, which is generally represented with higher brightness than that of a normal tissue in an ultrasound image, may be adopted to determine the second predetermined threshold. If the pixel having the brightness difference of a positive value, the absolute value of which is greater than the second predetermined threshold, is detected, then the extravasation detecting section 114a may be operable to output extravasation detection information. In such a case, the extravasation detection information may include at least one of icon, text, voice, image, color and alarm, although it is not limited thereto.

In yet another embodiment, the extravasation may be detected by using size variation of the blood vessel. FIG. 3 is a block diagram showing an illustrative embodiment of the processing unit 140. As shown in FIG. 3, the processing unit 140 may include an image forming section 141b. The image forming section 141 b may be operable to form first and second ultrasound images based on the first and second receive signals outputted from the Tx/Rx unit 110. The first ultrasound image may represent an image of the target object obtained before injecting the contrast agent, while the second ultrasound image may represent an image of the target object obtained after injecting the contrast agent. In another embodiment, the image forming section 141 b may be operable to consecutively form more than two ultrasound images based on multiple receive signals outputted from the Tx/Rx unit 110, which are obtained while injecting the contrast agent into the target object.

The processing unit 140 may further include a boundary detecting section 142b. In one embodiment, the boundary detecting section 142b may be operable to detect boundaries of the blood vessels on the first and second ultrasound images. In another embodiment, the boundary detecting section 142b may be operable to detect boundaries of the blood vessels on more than two ultrasound images, which are obtained while injecting the contrast agent. The boundary of the blood vessel may be detected based on variation of the pixel brightness determined by using a differential operator. In one embodiment, Sobel, Prewitt, Robert, Canny mask and the like may be adopted as a boundary mask. Also, the boundary of the blood vessel may be detected based on a difference of eigenvalues using a structure tensor.

The processing unit 140 may further include a size computing section 143b. The size computing section 143b may be operable to compute a size of the blood vessel based on the detected boundaries of the blood vessels. In one embodiment, the size of the blood vessel may be a diameter, a circumference and the like. In one embodiment, the size computing section 143b may be operable to compute a first size of the blood vessel based on the detected boundary of the blood vessel on the first ultrasound image and a second size of the blood vessel based on the detected boundary of the blood vessel on the second ultrasound image. In another embodiment, the size computing section 143b may be operable to sizes of the blood vessels based on the detected boundaries of the blood vessels on the plurality of ultrasound images. The size computing section 143b may be further operable to compute a difference of the sizes of the blood vessels between the first and second ultrasound images.

The processing unit 140 may further include an extravasation detecting unit 144b. The extravasation detecting section 144b may be operable to compare the size difference computed from the size computing section 143b with a third predetermined threshold. If the size difference is greater than the third predetermined threshold, then the extravasation detecting section 144b may be operable to output extravasation detection information. In one embodiment, the extravasation detection information may include an icon, a text, a voice, an image, a color or an alarm, although it is limited thereto.

Referring back to FIG. 1, the ultrasound system 100 may further include an output unit 150. The output unit 150 may be operable to output the extravasation detection information. Also, the out put unit 150 may be further operable to display the ultrasound images. The output unit 150 may include a display unit (not shown) and a sound output unit (not shown). The display unit may include a LCD monitor, a CRT monitor and the like. The sound output unit may include a speaker for outputting the voice and alarm.

As mentioned above, since the extravasation may be detected by using the ultrasound images, the operator may recognize the extravasation before the flesh swells up. Also, the extravasation may be detected at an early stage. Further, since the probe is fixed on the patient, the operator may freely use his or her hands.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a transmission/reception (Tx/Rx) unit configured to transmit an ultrasound beam to a target object and receive ultrasound echoes reflected from the target object to thereby output receive signals, wherein the receive signals include first receive signals obtained before injecting a fluid into the target object and second receive signals obtained after injecting the fluid into the target object; and
a processing unit configured to form a first ultrasound image based on the first receive signals and a second ultrasound image based on the second receive signal, the processing unit being further configured to compare the first ultrasound image and the second ultrasound image for detecting extravasation at the target object.

2. The ultrasound system of Claim 1, wherein the Tx/Rx unit includes:
a probe configured to transmit/receive the ultrasound beam; and
a fixing unit configured to fix the probe at a predetermined place on the target object.

3. The ultrasound system of Claim 2, wherein the processing unit includes:
an ultrasound image forming section configured to form a first ultrasound image based on the first receive signals and a second ultrasound image based on the second receive signals;
a pixel brightness detecting section configured to detect a brightness of pixels contained in the first and second ultrasound images;
a brightness difference computing section configured to compute a brightness difference of pixels positioned at identical locations between the first ultrasound image and the second ultrasound image; and
an extravasation detecting section configured to detect the extravasation based on the brightness difference to thereby output extravasation information.

4. The ultrasound system of Claim 3, wherein the extravasation detecting section is configured to output the extravasation information when the brightness difference has a negative value and an absolute value of the brightness difference is greater than a first predetermined threshold value.

5. The ultrasound system of Claim 3, wherein the extravasation detecting section is configured to output the extravasation information when the brightness difference has a positive value and is greater than a second predetermined threshold value.

6. The ultrasound system of Claim 2, wherein the processing unit includes:
an ultrasound image forming section configured to form a first ultrasound image based on the first receive signals and a second ultrasound image based on the second receive signals;
a boundary detecting section configured to detect boundaries of the target object in the first and second ultrasound images;
a size computing section configured to compute a first size of the target object on the first ultrasound image and a second size of the target object on the second ultrasound image based on the detected boundaries, the size computing section being further configured to compute a difference between the first size and the second size; and
an extravasation detecting section configured to detect the extravasation based on the size brightness difference to thereby output extravasation information.

7. The ultrasound system of Claim 6, wherein the extravasation detection section is configured to output the extravasation information when the size brightness difference is greater than a third predetermined threshold.

8. A method of detecting extravasation in an ultrasound system including a transmission/reception (Tx/Rx) unit and a processing unit, the method comprising:
using a transmission/reception (Tx/Rx) unit within the ultrasound system to transmit an ultrasound beam to a target object and receive ultrasound echoes reflected from the target object to thereby output receive signals, wherein the receive signals include first receive signals obtained before injecting a fluid into the target object and second receive signals obtained after injecting the fluid into the target object; and
using a processing unit within the ultrasound system to form a first ultrasound image based on the first receive signals and a second ultrasound image based on the second receive signals, and compare the first ultrasound image and the second ultrasound image for detecting extravasation at the target object.

9. The method of Claim 8, further comprising using a fixing unit to fix a probe included in the Tx/Rx unit over the target object.

10. The method of Claim 9, wherein the processing unit is used to:
form a first ultrasound image based on the first receive signals and a second ultrasound image based on the second receive signals;
detect a brightness of pixels contained in the first and second ultrasound images;
compute a brightness difference of pixels positioned at identical locations between the first ultrasound image and second ultrasound image; and
detect the extravasation based on the brightness difference to thereby output extravasation information.

11. The method of Claim 10, wherein the processing unit outputs the extravasation information when the brightness difference has a negative value and an absolute value of the brightness difference is greater than a first predetermined threshold value.

12. The method of Claim 10, wherein the processing unit outputs the extravasation information when the brightness difference has a positive value and is greater than a second predetermined threshold value.

13. The method of Claim 9, wherein the processing unit is used to:
form a first ultrasound image based on the first receive signals and a second ultrasound image based on the second receive signals;
detect boundaries of the target object in the first and second ultrasound images;
compute a first size of the target object on the first ultrasound image and a second size of the target object on the second ultrasound image based on the detected boundaries, and compute a difference between the first size and the second size; and
detect the extravasation based on the size brightness difference to thereby output extravasation information.

14. The method of Claim 13, wherein the processing unit outputs the extravasation information when the size brightness difference is greater than a third predetermined threshold.
